# EUROPEAN PATENT APPLICATION

(11) **EP 3 744 233 A1**
(43) Date of publication of application: **02.12.2020**
(21) Application number: 19176922.3
(22) Date of filing: 28.05.2019
(51) Int. Cl.: A61B 5/00, A61B 5/04

(54) **SENSOR SYSTEM FOR MONITORING A SEDATION OF A PATIENT**

(71) Applicant: Koninklijke Philips N.V., 5656 AG Eindhoven (NL)
(72) Inventor: JOHNSON, Mark Thomas, 5656 AE Eindhoven (NL); KOLEN, Alexander Franciscus, 5656 AE Eindhoven (NL); HELLE, Michael Günter, 5656 AE Eindhoven (NL); WEISS, Steffen, 5656 AE Eindhoven (NL); SISODIA, Rajendra Singh, 5656 AE Eindhoven (NL)
(74) Representative: de Haan, Poul Erik

(57) **Abstract**

A sensor system (100) for monitoring a sedation of a patient (200), thereby improving the sedation monitoring and reducing manual effort.

## Description

### FIELD OF THE INVENTION

The invention is directed to a sensor system for monitoring a sedation of a patient, a computer-implemented method for monitoring a sedation of a patient, a computer program element and a medical imaging device.

### BACKGROUND OF THE INVENTION

Sedative drugs are administered before or during a medical imaging examination when the patient feels anxious or when it is necessary to ensure that the acquired images have diagnostic quality, for example in pediatric examination. Moreover, some imaging approaches such as special magnetic resonance imaging (MRI) sequences rely on long image acquisition times that bear the risk of patient movement resulting in decreasing image quality.

The probability of patient movement during medical image acquisition can be decreased by increasing the dosage of sedative drugs or by giving an extra bolus before or during the scan. However, this may increase the risk of complications for the patient and may be economically disadvantageous.

In general it is desirable only to give more sedative drug if the level of sedation is observed to have decreased below the level required to successfully complete the next scan.
Another problem related to MRI is that the fact that the patient can only hardly be accessed from outside the scanner room as the patient is located right in the center of the magnet bore.

### SUMMARY OF THE INVENTION

With embodiments of the invention, an improved sensor system for monitoring a sedation of a patient is provided.

The invention is defined by the independent claims. Further embodiments and advantages of the present invention are incorporated in the dependent claims and the description.
Technical terms are used by the common sense. If a specific meaning is conveyed to certain terms, definitions of terms will be given in the following in the context of which the terms are used.

According to a first aspect of the invention a sensor system for monitoring a sedation of a patient is presented. The sensor system comprises a contact element, which comprises at least two electrodes. Furthermore, the sensor system comprises a stimulus generator, wherein the stimulus generator is configured for generating an electric pulse, for actuating at least one body part of the patient. Furthermore, the contact element is configured for being attached to at least one body part of the patient, such that the generated electric pulse is transmitted from the stimulus generator to the at least one body part of the patient via the contact element. In addition, the sensor system is configured for detecting a response of the at least one body part to the generated electric pulse. Furthermore, the sensor system is configured for generating a signal indicative for the sedation of the patient based on the detected response of the at least one body part of the patient.

This embodiment can provide the advantage that a sedation level of a patient can be monitored and in particular can be quantified with the help of the described sensor system. Therefore, no response has to be acquired by supervising personnel and therefore the required labor for medical imaging can be reduced. In addition, the assessment of the sedation of the patient can be quantified and qualified because the response of the patient to monitor the sedation is not evaluated by a human being which interprets the response but by the suggested sensor system and thereby the quality of the sedation monitoring can be improved.

In other words, a contact element can be provided which is attachable to a patient, in particular a body part of a patient, wherein the contact element can comprise at least two electrodes, which are able to transmit an electric pulse from a stimulus generator to the body part of the patient. The contact element will be further explained in the following.

The electric pulse can be configured in such a way that the electric pulse actuates the body part of the patient, for example, the electric pulse triggers a reaction of a nerve in the body part. The actuation of the at least on body part can be triggered by an electric pulse, which comprises a single and/or a plurality of electric pulses, which have a duration between 50 µs and 1000ms, a frequency of 1x10^-1 to 10^4 Hz and/or an interval between 0,1 Hz and 5 Hz. Alternatively or in addition, the electric pulse can be modulated between 0,5 µA and 20µA. Furthermore, the electric pulse can be altered between 0 and 100 V. This is further described with reference to preferred embodiments.

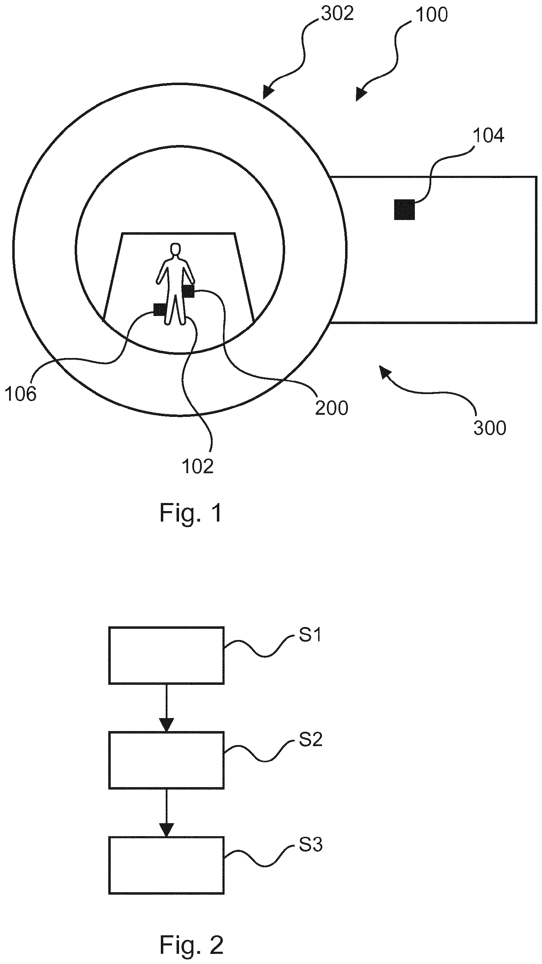

The sensor system can detect the response of the body part, for example, the response of a nerve due to the stimulation with the electric pulse and subsequently the sensor system can generate a signal indicative for the sedation of the patient based on the detected response. Furthermore, the sensor system can be configured for generating the signal subsequently to the detected response. This configuration of the sensor system is described in further detail in Fig. 1 and in the corresponding figure description.

In particular, the contact element and the stimulus generator can be a transcutaneous electrical nerve stimulus (TENS) which can be used in order to determine the sedation of the patient.

The contact element can be for example a patch, which can comprise the two electrodes of the contact element, wherein the patch can be attached to the body part of the patient. The contact element can be connected to the stimulus generator by a wired or wireless connection.

Furthermore, the stimulus generator can be configured to alter the electric pulse in terms of a waveform, amplitude, duration, frequency and/or pattern. The alteration of the electric pulse will be explained hereinafter in more detail. Furthermore, the stimulus generator can take into account the impedance/resistance generated by the contact element and the transition of the electric pulse between the body part and the contact element. The determined impedance can be used for selecting a position of the contact element or an electrode out of an array of electrodes where the impedance is the lowest such that the electric pulse is most efficient for actuating the at least one body part. This will be explained in more detail hereinafter.

In an exemplary embodiment, a contact element, which comprises the two electrodes, is attached to the patient at a body part, for example a foot or a toe. The stimulus generator can then generate an electric pulse, which is transferred via the contact element into the body part of the patient. The sensor system is capable of analyzing a response of the body part, in particular a nerve that is located in the foot, in response to this electric pulse. Based on the response of the body part, the sensor system can then generate a signal indicative for the level of sedation of the patient.

In an exemplary embodiment of the invention the at least one body part can comprise two body parts, wherein the first body part can be stimulated with the electric pulse of the stimulus generator, wherein the stimulation of the first body parts interacts with the second body part, thereby the sensor system is configured for detecting the response to the generated electric pulse on the second body part. In other words, the contact element can be attached to the foot of a patient and the electric pulse is transmitted to the foot but the response to the electric pulse can be detected by a facial expression of the patient. In addition, a further electrode can be attached to the second body part. The sensor system can detect the response to the electric pulse with the electrode attached to the second body part. In particular, the electrode can be a part of an electroencephalography (EEG) device and/or an electromyography (EMG) device. Moreover, the response of the actuation of the body part may be visible in another part of the body. For example, when triggering a heat sensation of a nerve in the foot may indeed cause the foot to move or the nerve to tighten the muscle without moving it (picked up by an EMG sensor). Additionally, the triggering of a nerve with the electric pulse could also result in a facial expression change (a grimace) or indeed even a response of the brain neurons, which can be picked up by an EEG sensor.

In an further exemplary embodiment of the invention the at least one body part can comprise three body parts, wherein the contact element is attachable to a first and a second body part of the patient, and wherein the sensor system can be configured for receiving an input indicative for an actuation of the first and/or the second body part via the third body part. In other words, the contact element can be attached to both feet of a patient and the response to the electric pulse can be seen in the face of the patient, for example a grimace. In order to assess - and in particular to quantify - a level of sedation of a patient TENS, as explained hereinbefore, can be used for providing a standardized, repeatable and quantifiable measure of the sedation status of a patient. TENS can be used for many consecutive threshold determinations at different points in time. A first threshold may serve as the reference of the sedation of the patient. The sedation of the patient can be known and/or a possibly correction of the initial depth of sedation can be performed. The subsequent thresholds may be measured at intervals of minutes later in time and compared to the reference of the sedation of the patient. If there is a large difference between thresholds, this indicates a change in sedation depth.

The response to the stimulus can be any of the known response assessment methods for example vocal, facial expression, movement, vital sign, brain activity, EMG as an assessment of muscle activity such as muscle stiffening or muscle activation and the like.

The present invention uses the fact that the presence of a sedative drug can effect the firing of the nerve induced by the TENS or the transmission of the nerve signal to the brain. As such, TENS can work with to assess the sedation level of a sedated patient. In case of pain and heat sensitive nerves, the pain and heat response would be similar to the response of a needle prick stimulus or a hot/cold water response, since this response can be assessed cognitively in the brain. Alternatively or in addition, when stimulating muscle activation nerves, the muscle activation can be a hard-wired response, since the activation does not pass through the cognitive system. However, even some drugs, such as aspirin, can slow down the muscle signals and so can sedatives.

In addition, TENS stimulus could assist a (semi) quantitative assessment of the sedation level of the patient. Whilst the threshold of response to the TENS signal may be different for individual patients, the TENS stimulus proposed can be able to show changes in the sedation state as a shift in a threshold, which is indicative for the point when the patient feels pain, heat, or EMG signal starts to rise, in particular even before muscle contraction.

In particular it can be chosen which type of sensory stimulation is performed, for example based on different scan types, it can be decided to have specific variants of TENS stimulus, for example for pain and heat stimulation or muscle stimulation.

Furthermore, a position of the contract element and/or the electrode can be adapted based on patient cognition, tolerance, sensitivity and/or the type of medical scan.

In another exemplary embodiment, the sensor system can be used during imaging, i.e. autonomous imaging. During autonomous imaging the stimulus can be able to induce a response in the patient without a muscle being activated otherwise this can lead to motion artefacts. Thus, the system presented herein is provided according to another exemplary embodiment together with a medical imaging device, e.g. an autonomous imaging device. This will be explained in more detail hereinafter.

Furthermore, the electric pulse, the TENS pulse shapes, can be adapted in order to induce a response in the patient without a muscle being activated. For example, the TENS waveforms (e.g. frequencies, durations, pulse widths and the like) can be tuned, such that pain and/or heat nerves can be stimulated but do not induce motion artefacts by triggering muscle activation. The following table comprises examples of the alteration of the electric pulse:

**Table 1: examples of alteration of the electric pulse**

| | |
|---|---|
| Electric pulse waveform | -Monophasic |
| | -Symmetrical biphasic |
| | -Asymmetrical biphasic |
| Electric pulse amplitude | 1-50 mA into 1kΩ load |
| Electric pulse duration | 10-1000µs |
| Electric pulse frequency | 1-250 Hz |
| Electric pulse pattern | -Continuous or burst |
| | -Modulated in: 1) frequency, 2) amplitude, 3) electric pulse duration, 4) random modulation |

The skilled reader will of course appreciated that these numbers are only examples and several other alterations of the electric pulse may be used.

Moreover, the sensor system can be configured for determining an actuation of the at least one body part of the patient and/or for receiving an input indicative for the actuation of the at least one body part of the patient. The input can be any response selected from the group consisting of vocal, facial expression, movement, vital sign, brain activity, and/or EMG.

According to an embodiment the sensor system comprises an interface element, which is configured for receiving an input of the patient for the response of the at least one body part of the patient. The just described input of the sensor system can be inputted via the interface element, thereby detecting the response of the at least one body part of the patient to the electric pulse.

According to a further embodiment, the stimulus generator is configured for generating a sequence of electric pulses specific for at least one body part.

This embodiment can have the advantage that the accuracy of monitoring the sedation of the patient can be improved, since the electric pulse sequence is adapted to the specific body part and/or the specific nerves in said body part. In other words, the sensor system can be configured to generate a sequence of electric pulses, in particular a sequence of specific patterns of electric pulses, which are adapted in their pulse waveform, pulse amplitude, pulse duration, pulse frequency and/or pulse pattern of a specific body part. In particular, the sequence of electric pulses can be adapted to a type of nerve, which is located in the body part. In addition, the sequence of electric pulses can be adapted in terms of an analgesic effect. For example, the sequence of electric pulses is configured to trigger a cellular level stimulation. In particular, the sequence of electric pulses can be adapted to a kind of the at least one body part of the patient, for example the sequence of the electric pulse can be adapted to a finger and/or a foot, as will be explained in more detail hereinafter.

According to an embodiment, the stimulus generator is configured for altering at least one parameter of the electric pulse over a time period upon the generation of the electric pulse. Furthermore, the sensor system is configured for detecting an activation threshold value of the at least one body part by detecting the response of the body part over said time period and/or over a plurality of said time periods.

The detection of the activation threshold value can have the advantage, that the amount of sedative can be further reduced since the sedation of the patient can be estimated more precisely.

In other words, the sensor system can be further configured for altering the electric pulse in at least one parameter wherein the at least one parameter of the electric pulse can be a waveform, an amplitude, a duration, a frequency and/or a pulse pattern. In other words, the electric pulse can be formed over the time period in which the parameters of the electric pulse are changed. The changing/modulating of the electric pulse can then be repeated with the same change of the parameters in each time period of the electric pulse to form a sequence of the changing electric pulses. Furthermore, the sensor system can detect an activation threshold value within a single time period, over the plurality of time periods and/or for each of the time periods. Based on the electric pulse, the sensor system can detect the activation threshold value, which describes the response of the body part, in particular a nerve within the body part, to the generated electric pulse. In an exemplary embodiment, the system is configured to increase the pulse amplitude over time until a response of the nerve within the body part can be detected. Based on the shift of the activation threshold value over time the sedation of the patient can be estimated by the sensor system.

According to an embodiment, the parameter of the electric pulse, which is altered over time by the stimulus generator, is a waveform, an amplitude, a duration, a frequency and/or a pattern.

Also other parameters of the pulse may be changed in addition thereto.

This embodiment can have the advantage that by altering the parameters of the electrical pulse the quality of the sedation monitoring can be improved since different aspects, for example a transition impedance between the stimulus generator and the body part, can be accounted in the sedation monitoring of the patient.

For example, the pulse waveform can be monophasic, symmetrical biphasic and/or asymmetrical biphasic. The pulse amplitude can be for example between 0.1 and 100 mA into 0.5 kΩ to 5 kΩ load. The pulse duration can be between 1 ms and 10000 ms. In addition, the pulse frequency can be between 0.1 and 500 Hz. The pulse pattern can be modified into a continuous or a burst pattern and in addition can be modulated in frequency, in amplitude, in pulse duration and/or random modulation.

According to a further embodiment, the sensor system is configured for detecting a shift of the activation threshold over the time period and/or over the plurality of said time periods. In addition, the sensor system is configured for generating the signal based on the detected shift of the activation threshold value over the time period and/or over the plurality of said time periods.

This embodiment can have the advantage that by detecting the shift of the threshold value over time, the sedation monitoring is relative to the specific body part and therefore the sensor system can be used on any kind of body part of any patient since only the shift is estimated and not necessarily absolute values. Therefore, the quality of the sedation monitoring can be further improved.

In other words, the response of the body part or the nerve in the body part due to the electrical pulse is recorded over time. The electric pulse can be recorded for a single time period, for example for a duration in which the electric pulse is changed. Alternatively or in addition, the electric pules can be recorded for a plurality of time periods, for examples over a sequence of changes of the electric pulse. The shift can be determined within each of the time periods, over all of the plurality of the time periods and/or over sections of the plurality of the time periods. By evaluating the response, in particular the activation threshold value over a single time period and/or over a plurality of time periods, a shift of the activation threshold value can be detected. Based on said shift, the generation of the signal indicative for the sedation of the patient, can be generated in order to be able to inform supervising personnel whether a further sedation of the patient is needed or not.

According to an embodiment, the sensor system comprises a neurological sensor unit. In addition, this neurological sensor unit is configured for determining the actuation of the at least one body part of the patient.

This embodiment can have the advantage that the sensor system can evaluate the response of the body part to the electrical pulse with the help of the neurological sensor unit. Thereby, the personal effort of the supervising personnel is reduced since the supervising personnel does not need to communicate with the patient within the medical imaging device in order to monitor the sedation of the patient.

In other words, the neurological sensor unit can comprise an EMG device, which is capable to supervise the electrical activity produced by the nerves and/or muscles of the body part. The EMG device can use the contact element with the two electrodes or provide further contact elements or electrodes to monitor the response of the body part due to the actuation by the electric pulse.

According to an embodiment, the electric pulse is configured for actuating a pain and/or a heat-sensitive nerve of the at least one body part of the patient.

This embodiment can have the advantage that by actuating a pain or a heat-sensitive nerve of the at least one body part, no actuating of a muscle or a muscle-related nerve takes place and therefore no motion is triggered which could be reducing the quality of the medical imaging procedure.

In an exemplary embodiment, the pain sensitive nerve is an Aδ nerve, which has a diameter of about 1 to 4 µm and a conjunction velocity of 5 to 15 m/s and a duration of actuation peak of about 0.6 to 1.0 ms. In addition, a heat-sensitive nerve can be for example a fibre type C nerve which has a diameter of about 0.2 to 1.0 µm and a conjunction velocity of 0.2 to 2 m/s and furthermore a duration of action peak of 2.0 ms. The electric pulse generated by the stimulus generator can be configured for actuating a Aδ and/or C to be able to activate the just presented types of nerves. The following table shows examples of nerves, in particular fibre types, within the body part of the patient which can be actuated by the electric pulse:

**Table 2: Examples of nerves which can be actuated by the electric pulse**

| fibre type | diam. (µm) | Conduction velocity (m/s) | duration of action peak (ms) | function |
|---|---|---|---|---|
| Aα | 13-22 | 70-120 | 0.4-0.5 | motor, sensory (muscle sensors) |
| Aβ | 8-13 | 40-70 | 0.4-0.6 | touch, kinaesthesia |
| Aγ | 4-8 | 15-40 | 0.5-0.7 | efferent intrafusal fibres of muscle, touch |
| Aδ | 1-4 | 5-15 | 0.6-1.0 | fast pain, temperature, pressure |
| B | 1-3 | 3-14 | 1.2 | preganglionic |
| C | 0.2-1.0 | 0.2-2 | 2.0 | slow pain, temp, pressure, postganglionic |

Furthermore, muscle motion can be avoided by selecting a body part of the patient without too many muscle activation nerves. In addition a body part can be selected, which comprises many pain and thermal sensitive nerves. The TENS stimulus of the pain and thermal sensitive nerves could even be placed outside the scanner region. For example, the TENS electrodes can be attached under the foot and/or on the fingers of the patient.

According to an embodiment, the electric pulse is configured for actuating a muscle activation nerve of the at least one body part of the patient.

This embodiment can have the advantage that muscle activation nerves can be located easily and can be actuated by an electric pulse signal, thereby enabling the sensor system, in particular the contact element, to be attached at various body regions.

In particular, the electric pulse is configured on actuating a muscle nerve such that the contraction of the muscle is not triggered. An example for a muscle activation nerve can be an Aα fibre nerve, which has a diameter of about 13 to 22 µm and a conjunction velocity of 70 to 120 m/s as well as a duration of action peak between 0.4 and 0.5 ms. Other examples are provided by table 2 as shown above.

According to an embodiment, the electric pulse is configured for tensing a muscle of the at least one body part of the patient, in particular without contracting the muscle of the at least one body part of the patient.

This embodiment can have the advantage that the quality of the medical imaging is improved since a movement of the muscle is prohibited since the electric pulse is adapted to the type of nerve and therefore no motion artefacts appear on the medical image.

For example, the contact element is attachable to a body part and also an EMG is attachable to said body part. The stimulus generator generates an electric pulse monitored by the EMG such that only a tensioning of the muscle occurs and no contraction of the muscle. Therefore, a sedation monitoring can take place without generating motion artefacts in the medical image. In other words, it can be made use of a muscle based stimulus response using TENS to activate muscle activation nerves and EMG as response measurement. With the help of the EMG the response can be measured before a muscle contraction occurs, in particular the muscles stiffens without a contraction.

Alternatively or in addition, the sensor system can be capable of detecting a localized muscle activation, which may lead to a muscle contraction such that the response to the stimulus is still localized. For example, the contact element is located on feet as the patient will move its feet due to the electric pulse and the movement may not affect the scan quality, since the scan is directed to the head of the patient. The system in this embodiment is thus capable of detecting feet responses and evaluate them for the signal generation as described before and hereinafter.

In another example, the sensor system can trigger the TENS stimulus in between scans. This way, the stimulus need not be very fine grained and based on the response, the qualification can be done for subsequent scans and probability of patient disruptive events can be estimated.
According to an embodiment, the sensor system is configured for determining an electrical impedance of the at least one body part via the contact element.

This embodiment can have the advantage that a type and/or a position of nerve can be identified by determining the electrical impedance/resistance of the at least one body part, the sedation monitoring can be further improved and the quality of the medical imaging acquisition can be further improved.

According to an exemplary embodiment the contact element comprises an array of electrodes, wherein the array of electrodes is attachable to the at least one body part, wherein the sensor system is configured for determining the impedance/resistance for each pair of electrodes of the array and/or for each electrode in combination with a reference electrode of the array of the at least one body part. In other words, the array of electrodes can be attached to the at least one body part. Then, the sensor system can determine the impedance of each pair of the electrodes of the array of electrodes. Alternatively or in addition, the array can comprise a reference electrode, wherein the impedance can be measured between the reference electrode and each of the other electrodes of the array. The sensor system can then select the pair of electrodes, which has the lowest impedance/resistance, such that the electric pulse can be most efficient for actuating the at least one body part. Furthermore, the array of electrodes can be attached to two or more body parts, for example at both feet of the patient.

In an exemplary embodiment, a contact element and an EMG, in particular at least one electrode of the EMG, is attached to a body part of the patient, wherein the at least one electrode of the EMG in combination with the contact element can measure the electric impedance of the at least one body part. In case the body part could be identified, the electrical pulse can be adapted in respect to the corresponding body part. In particular, the electric impedance of the at least one body part of the patient can be used for determining a type and/or a position of a nerve within the body part.

In an exemplary embodiment, the electric pulse can be definable by a user in terms of waveform, amplitude, duration, frequency and/or pattern. In addition, the sensor system can comprise a selection unit on which a user may choose between a plurality of different body parts. Furthermore, the stimulus generator can be configured for adapting the waveform, the amplitude, the duration, the frequency and/or the pattern of the electric pulse based on a received choice of the user regarding the body part. This embodiment can have the advantage that the reliability of the sensor system is improved, since the supervising personal can select the body parts and the corresponding electric pulse sequences, thereby reducing the risk of mishandling.

In a further embodiment, the electric pulse can be adapted based on a kind of the medical imaging procedure. For example, the sensor system can be used in combination with a MRI system, the electric pulse and/or the electric pulse sequence can be adapted to the MRI application and/or MRI environment. On the other hand, the electric pulse can be used in combination with a computer tomography (CT) system. The electric pulse and/or the sequence of electric pulses can be adapted to the CT application and/or CT environment.

In a further embodiment, the at least one body part of the patient can be a foot and/or a finger. In addition, the body part of the patient can be any body part, which is remote of the region of interest of the medical imaging procedure.

In an exemplary embodiment the electric pulse is configured for not contracting the at least one body part of the patient. In other words, the electric pulse does not trigger a contraction of the muscle, but a stiffing or any form of response, which can be measured with the help of an EMG system.

In a further embodiment, the at least one body part of the patient is positioned outside of a medical imaging device. Thus, it is provided in proximity of the imaging device. In case a body part of the patient is located remote of a medical imaging device, which can be used in combination with the sensor system, the contact element, can be contacted to the body part of the patient, which is located outside of the medical imaging device.

According to a second aspect of the invention, a computer-implemented method for monitoring a sedation of a patient during a medical imaging procedure comprises the step of generating an electric pulse, which is configured for actuating on at least one body part of the patient. Furthermore, the method comprises the step of detecting a response of the at least one body part of the patient and generating a signal indicative for the sedation of the patient based on the detected response of the at least one body part of the patient.

This method can have the advantage that a sedation monitoring of a patient can be improved since an electrical pulse is generated to actuate at least a body part of the patient such that the electric pulse triggers a response of the at least one body part to the electrical pulse which can be detected. Furthermore, the detected response can lead to a generating of a signal indicative for the sedation of the patient. In an exemplary embodiment, an electrical pulse is generated by the stimulus generator wherein the electrical pulse is configured to actuate the at least one body part of a patient, in particular a nerve of the body part. The body part reacts on the electric pulse such that a response can be thereby detected. Furthermore, a signal can be generated which is based on the detected response of the at least one body part due to the electric pulse such that a signal indicative for the sedation can be generated or outputted.

Features and elements of the sensor system as described herein can be features and elements and/or steps of the method as described herein and the other way round.

A further aspect of the invention is a computer program element, which, when executed on a processor unit, instructs the processor unit to perform the method as described herein.

The computer program element may be part of a computer program, but it can also be an entire program by itself. For example, the computer program element may be used to update an already existing computer program to get to the present invention.

A further aspect of the invention is a computer-readable medium, which stores the computer program element as described herein.

The computer readable medium maybe seen as a storage medium, such as for example, a USB stick, a CD, a DVD, a data storage device, a server, a hard disk, or any other medium on which a program element as described above can be stored.

Another aspect of the invention is a medical imaging device, which comprises a medical imaging acquisition device, which is configured for generating a medical image of at least one body part of a patient. Furthermore, the medical imaging device comprises a sensor system as described herein.

This embodiment can have the advantage that the amount of sedation is reduced since with the help of the sensor system, the sedation of the patient can be estimated way more precisely and therefore the imaging quality can be further improved. The medical imaging device can be for example, an MRI, a CT, an X-ray and/or a positron emission tomography (PET). In addition, the medical imaging device can be any combination of the just listed examples, like a PET-CT. In particular, the medical imaging device comprises a patient table, on which the patient can be examined.

According to an embodiment, the medical imaging device is further configured for first generating a first image of the at least one body part. Furthermore, the medical imaging acquisition device is configured for second subsequently generating the signal indicative of the response of the at least one body part. The medical imaging acquisition device is further configured for third subsequently generating a second image of the at least one body part.

This embodiment can have the advantage that the monitoring of the sedation of the patient does not interfere the medical image acquisition. For example, a first sequence of medical images, for example MRT images, are acquired. Subsequently, after the first sequence of images is finished, the sedation of the patient can be estimated with the help of the sensor system. In case the sedation of the patient is sufficient for acquiring a second sequence of medical images, the second image of the at least one body part is generated with the help of the medical image acquisition device.

According to a further embodiment, the contact element of the sensor system is integrated at least partially in the medical imaging device, thereby the contact element contacts the at least one body part of the patient.

This embodiment can have the advantage that no contact element has to be attached to the patient, since the contact element is already integrated in a part of the medical imaging device, for example the patient table or the patient's headrest, such that the sedation of the patient can be monitored.

The contact element and/or TENS can use adhesive patch electrodes but in the setting of autonomous imaging it may beneficial to avoid the work step to manually apply electrodes to the skin. Therefore, the contact element and/or the electrodes, e.g. the TENS electrodes, may be integrated into the imaging devices such that they automatically make contact to the skin upon positioning of the patient. The electrodes may be integrated into the patient support, for example in areas where typically bare skin is located, e.g. hands. The electrodes may also be integrated in addition to the patient support as arm rests or the head rest. For example, in MR examinations, electrodes may also be integrated into surface RF coil arrays used for MR imaging or into ear muffs used for ear protection.

According to a further embodiment, the contact element is integrated in a patient support, in an arm rest, in a head rest, in a surface RF coil array, and/or an ear muff of the medical imaging device. In other words, the electrodes of the contact element of the sensor system can be integrated in the medical imaging device such that the electrodes of the contact element establish a contact between the electrodes and the at least one body part of the patient.

All disclosures as described herein in relation to any aspect of the invention applies equally to all other aspects of the invention.

In the following, examples and embodiments of the invention are described with reference to the figures.

### BRIEF DESCRIPTION OF THE DRAWINGS

Fig. 1 shows a medical imaging acquisition device according to an embodiment.
Fig. 2 shows a flow-chart illustrating the steps of the method according to an embodiment.

The reference symbols used in the drawings, and their meanings, are listed in a summary form in the list of reference symbols. In principle, identical parts are provided with the same reference symbols in the figures.

### DETAILED DESCRIPTION OF EMBODIMENTS

Fig. 1 shows a medical image device 300 according to an embodiment. The sensor system 100 for monitoring a sedation of a patient 200 comprises a contact element 102, which comprises at least two electrodes. Furthermore, the sensor system 100 comprises a stimulus generator 104, wherein the stimulus generator 104 is configured for generating an electric pulse, for actuating at least one body part of the patient 200. Furthermore, the contact element 102 is configured for being attached to at least one body part of the patient 200 such that the generated electric pulse is transmitted from the stimulus generator 104 to the at least one body part of the patient 200 and the contact element 102. In addition, the sensor system 100 is configured for detecting a response of the at least one body part to the generated electric pulse. Moreover, the sensor system 100 is configured for generating a signal indicative for the sedation of the patient 200 based on the detected response of the at least one body part of the patient 200.

The embodiment of the medical image device 300 shown in Fig. 1 can advantageously comprise a sensor system 100. The patient table of the medical image device 300 can comprise at least one electrode of the contact element 102. Alternatively or in addition the patient table can comprise an electrode of the neurological sensor unit 106. The electrodes of the contact element 102 and the neurological sensor unit 106 can form a contact with the patient 200 without the need of attaching manually patches to the patient 200. The medical image device 300 can comprise the stimulus generator 104. The stimulus generator 104 can generate an electric pulse, which is introduced into the patient 200 via the contact element 102. The electric pulse is configured for actuating at least one body part of the patient 200, in particular without generating any motion artefacts during the generating of medical images with the help of the medical image acquisition device 302. The sensor system 100 can be configured to detect, in particular with the help of the neurological sensor unit 106, a response of the body part of the patient 200 to the electric pulse generated by the stimulus generator. The sensor system 100 can calculate a level of sedation of the patient 200 based on the detected response of the body part of the patient 200 and generate a signal, which is indicative for the sedation of the patient 200.

This embodiment can have the advantage that a sedation of the patient 200 is detected and monitored more precisely, such that an amount of sedative can be reduced in order to be able to acquire proper medical images with help of the medical image device 300.

As can be seen in the Fig. 1, the medical imaging device 300 comprises a medical imaging acquisition device 302 in which the patient 200 can be located. Furthermore, the medical imaging device can comprise a sensor system 100, which has a stimulus generator 104 and a contact element 102. The contact element 102 can be contacted to the patient 200. The sensor system 100 can further comprise a neurological sensor unit 106, which is capable to detect the response of the at least one body part of the patient 200 to the electric pulse, which has generated by the stimulus generator 104.

Fig. 2 shows a flow-chart illustrating the method according to an embodiment. The method comprises the step of generating S1 an electric pulse, which is configured for actuating at least one body part of the patient 200. Furthermore, the method comprises the step of detecting S2 a response of the at least one body part of the patient 200. In addition, the method comprises the step of generating S3 a signal indicative for the sedation of the patient 200 based on the detected response of the at least one body part of the patient 200.

This embodiment can have the advantage that the personal effort of a supervising personnel can be reduced since the supervising personnel does not need to determine the sedation of the patient.

Where an indefinite or definite article is used when referring to a singular noun, e.g. "a", "an" or "the", this includes a plurality of that noun unless something else is specifically stated. The terms "about" or "approximately" in the context of the present invention denote an interval of accuracy that the person skilled in the art will understand to still ensure the technical effect of the feature in question.

Furthermore, the terms "first", "second", "third" or "(a)", "(b)", "(c)", "(d)" or "(i)", "(ii)", "(iii)", "(iv)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

### LIST OF REFERENCE SIGNS:

100 - sensor system
102 - contact element
104 - stimulus generator
106 - neurological sensor unit
200 - patient
300 - medical imaging device
302 - medical imaging acquisition device
S1 - generating an electric pulse
S2 - detecting a response
S3 - generating a signal

## Claims

1. A sensor system (100) for monitoring a sedation of a patient (200), wherein the sensor system (100) comprises:
a contact element (102), which comprises at least two electrodes,
a stimulus generator (104),
wherein the stimulus generator (104) is configured for generating an electric pulse, for actuating at least one body part of the patient (200),
wherein the contact element (102) is configured for being attached to the at least one body part of the patient (200) such that the generated electric pulse is transmittable from the stimulus generator (104) to the at least one body part of the patient (200) via the contact element (102),
wherein the sensor system (100) is configured for detecting a response of the at least one body part to the generated electric pulse, and
wherein the sensor system (100) is configured for generating a signal indicative for the sedation of the patient (200) based on the detected response of the at least one body part of the patient (200).

2. The sensor system according to claim 1,
wherein the stimulus generator (104) is configured for generating a sequence of electric pulses specific for the at least one body part.

3. The sensor system according to any of the preceding claims,
wherein the stimulus generator (104) is configured for altering at least one parameter of the electric pulse over a time period upon the generation of the electric pulse, and
wherein the sensor system (100) is configured for detecting an activation threshold value of the at least one body part by detecting the response of the body part over said time period and/or over a plurality of said time periods.

4. The sensor system according to claim 3,
wherein the parameter of the electric pulse, which is altered over time by the stimulus generator (104) is a waveform, an amplitude, a duration, a frequency, and/or a pattern.

5. The sensor system according to any of claims 3 to 4,
wherein the sensor system (100) is configured for detecting a shift of the activation threshold value over the time period and/or over the plurality of said time periods, and
wherein the sensor system (100) is configured for generating the signal based on the detected shift of the activation threshold value over the time period and/or over the plurality of said time periods.

6. The sensor system according to any of the preceding claims,
wherein the sensor system (100) comprises a neurological sensor unit (106), and
wherein the neurological sensor unit (106) is configured for determining the actuation of the least one body part of the patient (200).

7. The sensor system according to any of the preceding claims,
wherein the electric pulse is configured for actuating a pain and/or a heat sensitive nerve of the at least one body part of the patient (200).

8. The sensor system according to any of the preceding claims,
wherein the electric pulse is configured for actuating a muscle activation nerve of the at least one body part of the patient (200).

9. The sensor system according to claim 8,
wherein the electric pulse is configured for tensing a muscle of the at least one body part of the patient (200), in particular without contracting the muscle of the at least one body part of the patient (200).

10. The sensor system according to any of the preceding claims,
wherein in the sensor system (100) is configured for determining an electrical impedance of the at least one body part via the contact element (102).

11. A computer implemented method for monitoring a sedation of a patient (200) during a medical imaging procedure, comprising the steps of:
- generating (S1) an electric pulse, which is configured for actuating on at least one body part of the patient (200),
- detecting (S2) a response of the at least one body part of the patient (200),
- generating (S3) a signal indicative for the sedation of the patient (200) based on the detected response of the at least one body part of the patient (200).

12. A computer program element, which when executed on a processor unit, instructs the processor unit to perform the steps of the method according to claim 11.

13. A medical imaging (300) device, comprising:
- a medical image acquisition (302) device (302), which is configured for generating a medical image of at least one body part of a patient (200),
- a sensor system (100) according to any of the claims 1 to 10.

14. The medical imaging device (300) according to claim 13,
wherein medical image acquisition (302) device is configured for:
- first generating a first image of the at least one body part,
- second subsequently generating the signal indicative of the response of the at least one body part, and
- third subsequently generating a second image of the at least one body part.

15. The medical imaging device according to claims 13 to 14,
wherein the contact element (102) of the sensor system (100) is integrated at least partially in the medical imaging device (300), such that the contact element (102) contacts the at least one body part of the patient (200).
